# EUROPEAN PATENT SPECIFICATION

(11) **EP 4 417 129 B1**
(45) Date of publication and mention of the grant of the patent: **29.07.2026**
(21) Application number: 22903835.1
(22) Date of filing: 04.10.2022
(51) Int. Cl.: A61B 5/37, A61B 5/055, A61B 5/384

(54) **INFORMATION PROCESSING DEVICE FOR DIAGNOSIS OF EPILEPSY, INTRAVASCULAR DEVICE, INFORMATION PROCESSING METHOD FOR DIAGNOSIS OF EPILEPSY, AND COMPUTER PROGRAM FOR DIAGNOSIS OF EPILEPSY**
INFORMATIONSVERARBEITUNGSVORRICHTUNG ZUR DIAGNOSE VON EPILEPSIE, INTRAVASKULÄRE VORRICHTUNG, INFORMATIONSVERARBEITUNGSVERFAHREN ZUR DIAGNOSE VON EPILEPSIE UND COMPUTERPROGRAMM ZUR DIAGNOSE VON EPILEPSIE
DISPOSITIF DE TRAITEMENT D'INFORMATIONS POUR LE DIAGNOSTIC DE L'ÉPILEPSIE, DISPOSITIF INTRAVASCULAIRE, PROCÉDÉ DE TRAITEMENT D'INFORMATIONS POUR LE DIAGNOSTIC DE L'ÉPILEPSIE ET PROGRAMME INFORMATIQUE POUR LE DIAGNOSTIC DE L'ÉPILEPSIE

(30) Priority: 09.12.2021 JP 2021199978
(43) Date of publication of application: 21.08.2024
(60) Divisional application: 26152056.3
(73) Proprietor: Epsilon Medical Inc., Tokyo 103-0024 (JP)
(72) Inventor: MATSUMARU, Yuji, Tokyo 103-0023 (JP); ISHIDA, Hiroki, Tokyo 103-0023 (JP)
(74) Representative: Ström & Gulliksson AB
(86) International application number: PCT/JP2022/037157
(87) International publication number: WO 2023/105901

(56) References cited:
- JP-A- 2002 360 538
- JP-A- 2007 111 118
- JP-A- 2008 188 428
- JP-A- 2013 111 422
- JP-A- 2016 528 002
- JP-A- 2020 192 327
- US-A1- 2012 314 919
- US-A1- 2016 120 457
- US-A1- 2020 016 396
- US-A1- 2020 029 849

## Description

### TECHNICAL FIELD

The present invention relates to an information processing machine , method and computer program for use in diagnosis of epilepsy.

### BACKGROUND ART

In medical operations for refractory epilepsy (epilepsy where there is no mitigation of seizures and symptoms observed by using medicines), for example, such medical operations have been performed where a part of brain tissue is surgically cut. Various methods have been used to identify a site to be cut, but each method has its own challenges. For example, an electrode has been applied onto a scalp to measure brain waves. Although measuring brain waves as described above is not invasive, and is possible to implement in many facilities, spatial resolution and time resolution are low, and it may not be possible to correctly identify a site that should be cut.

Furthermore, for example, a subdural electrode has been applied and an electrode for depth electroencephalogram has been inserted after performing a craniotomy to measure brain waves. Measuring brain waves as described above is highly invasive, while spatial resolution and time resolution are high, and such intracranial electrodes as described above may result in disease complications. Furthermore, there have been other issues such as it is impossible to place such intracranial electrodes as described above for a long period of time depending on a state of a brain surface and there is only a small number of special medical doctors for neurosurgical operations, resulting in a limited number of facilities in which it is possible to implement such operations.

In recent years, such a technique has been disclosed that provides an electrode in a blood vessel and causes the electrode to sense, in the blood vessel, electrical activity in a part of nerve tissue. Patent Document 1 discloses a technique that causes a stent provided with an electrode to expand in a brain blood vessel, to be put in place on a wall of the blood vessel, and to sense electrical activity in a nearby part of nerve tissue.

### Citation List

### Patent Document

Patent Document 1: Japanese Unexamined Patent Application, Publication No. 2017-159079

US2012314919 A1 relates to a system and method for surgical planning for implanting a stimulation leadwire, registration of images for generating a model via which to determine how to apply a stimulation, for programming stimulation settings, and/or for applying a therapeutic stimulation, and/or for integration of components providing such functionality.

US2020016396 A1 relates to systems and methods for implanting electrodes in blood vessels in and/or outside the brain.

### DISCLOSURE OF THE INVENTION

### Problems to be Solved by the Invention

Note herein that there is an issue of, to identify a focal point of epilepsy and detect a seizure of epilepsy, how to select a position in a blood vessel for receiving a signal pertaining to an identified part of brain tissue.

In view of the situations described above, an object of the present invention is to provide a technique for properly sensing electrical activity in the brain tissue in diagnosing epilepsy.

### Means for Solving the Problems

To achieve the object described above, an aspect of the present invention is directed to an information processing machine according to claim 1.

### Effects of the Invention

According to the present invention, it is possible to provide a technique for properly sensing electrical activity in the brain tissue in diagnosing epilepsy.

### BRIEF DESCRIPTION OF THE DRAWINGS

Fig. 1 is a diagram illustrating an overview of image display;
Fig. 2 is a diagram illustrating an outline of a configuration of a system;
Fig. 3 is a block diagram illustrating a hardware configuration of a server;
Fig. 4 is a functional block diagram illustrating an example of a functional configuration of the server;
Fig. 5 is a flowchart illustrating an example of operation of image display processing;
Fig. 6 is a diagram illustrating examples of association tables;
Fig. 7 is a flowchart illustrating another example of operation of the image display processing;
Fig. 8 is a flowchart illustrating an example of operation of brain wave analysis processing; and
Fig. 9 is a diagram illustrating blood vessels in a brain.

### PREFERRED MODE FOR CARRYING OUT THE INVENTION

### (Embodiment)

### <Overview>

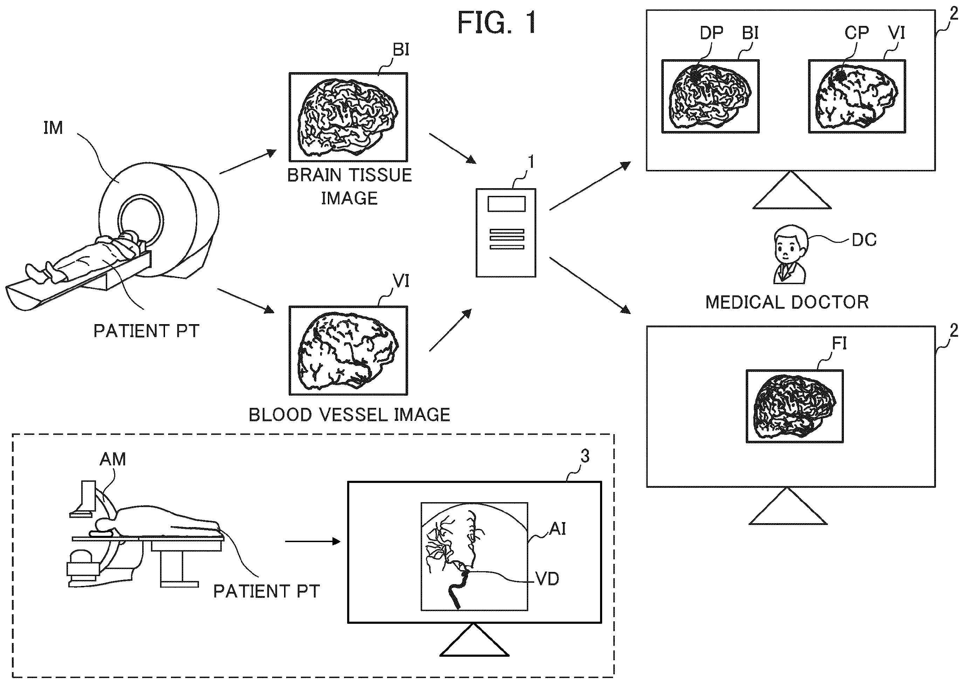

An embodiment will now be described herein with reference to the accompanying drawings. Fig. 1 is a diagram illustrating an overview of image display to which a server 1 (an information processing machine) according to the present embodiment is applied.

In the example illustrated in Fig. 1, the server 1 acquires various types of images from an image acquisition machine IM. The image acquisition machine IM is a machine that acquires an image indicating information of inside of a biological body in a patient PT (a subject and an organism such as an animal or a human being). More specifically, the image acquisition machine IM acquires, as information of inside of a biological body, an image (a first image) indicating biological tissue that generates an electric signal and a blood vessel image (a second image) indicating blood vessels in or near the biological tissue. The biological tissue includes brain tissue. That is, the server 1 acquires, from the image acquisition machine IM, a brain tissue image BI (a first image) indicating brain tissue in the patient PT and a blood vessel image VI (a second image).

The brain tissue image BI is, for example, an image of the brain tissue, which is acquired by using magnetic resonance imaging (MRI). Specifically, the brain tissue image BI is an image acquired by utilizing a nuclear magnetic resonance phenomenon to capture tomographic images of the brain tissue, and is tomographic images or a three-dimensional image based on the tomographic images. It is possible, with the brain tissue image BI, to view a target, which is the brain tissue, at various angles. A medical doctor DC is able to understand, with the brain tissue image BI, a shape and a dimension of the brain tissue and a state of the brain tissue (for example, whether or not there is a tumor), for example.

The blood vessel image VI is, for example, a magnetic resonance image (MRA) of blood vessels, which is acquired by using the MRI described above. It is possible, with the blood vessel image VI, to view a target, which is a blood vessel (a blood stream), at various angles. The medical doctor DC is able to understand, with the blood vessel image VI, blood streams in the head, thicknesses of the blood vessels, and states of the blood vessels (for example, whether or not there is a tumor), for example.

The server 1 causes a terminal 2 (a display unit of the terminal 2 and a predetermined display unit) that the medical doctor DC possesses to display the acquired brain tissue image BI and the acquired blood vessel image VI. In the present embodiment, the server 1 performs two types of display in accordance with a setting of a position identification mode (a predetermined mode). For example, the server 1 causes, when the position identification mode is ON, the terminal 2 to display a brain tissue image BI and a blood vessel image VI in parallel to each other. At this time, the server 1 receives identification of a site (hereinafter referred to as a first site DP.) that is a target from which an electric signal is to be detected in the brain tissue. Then, the server 1 identifies a site corresponding to the first site DP described above, that is, a site (hereinafter referred to as a second site CP.) that is suitable for a device to be disposed in the blood vessel, among the blood vessels, to detect an electric signal from the first site DP described above. Furthermore, the server 1 causes the terminal 2 to display the identified second site CP. Furthermore, for example, the server 1 associates, when the position identification mode is OFF, the brain tissue image BI and the blood vessel image VI with each other. Then, the server 1 causes the terminal 2 to display the brain tissue image BI and the blood vessel image VI in an overlapped manner (in a synthesized manner), as a synthesized image FI (a third image). A method for associating a brain tissue image BI and a blood vessel image VI with each other will be described later.

The medical doctor DC refers to the brain tissue image BI and the blood vessel image VI or the synthesized image FI displayed on the terminal 2 to confirm a position (a position in the blood vessel) in which an intravascular device (not illustrated, an epilepsy diagnosis device) is to be disposed. Then, the medical doctor DC delivers the intravascular device into the brain blood vessel in the patient PT in a state where the device is interpolated in a catheter used for a medical operation in the brain blood vessel. At this time, the medical doctor DC causes, as illustrated in Fig. 1, an angio image AI (a real-time image and a fourth image) acquired with a blood vessel contrast-imaging examination AM (angiography) to be displayed on the display unit of the terminal 2 or another display machine (for example, an external display machine 3 illustrated in Fig. 1), refers to the angio image AI, and delivers the intravascular device into the brain blood vessel in the patient PT. Specifically, the medical doctor DC disposes the intravascular device at the second site CP displayed on the terminal 2, uses the intravascular device that is placed, and performs detection of an electric signal from the first site DP. Note that a number of the intravascular devices to be inserted may be one or plural. Note that the angio image AI is an image indicating a position of an intravascular device together with blood streams. For example, a position indicated by a symbol VD in the angio image AI illustrated in Fig. 1 represents a current position (for example, a position of a tip) of the intravascular device in the blood vessel. The medical doctor DC views the angio image AI (the fourth image) described above together with the synthesized image FI (the third image) described above, making it possible to easily and correctly deliver the intravascular device at the second site. Note that association of a brain tissue image BI, a blood vessel image VI, a synthesized image FI, and an angio image AI with each other may be optional, and the images may be or may not be associated with each other. Note that an angio image AI may be solely displayed, or may be displayed together with at least one image among a brain tissue image BI, a blood vessel image VI, and a synthesized image FI in parallel to each other.

The intravascular device is inserted into a brain blood vessel via a catheter used for a medical operation in the brain blood vessel. The intravascular device is disposed in a blood vessel in an organism, and includes at least one electrode for detecting activity in a part of the biological tissue, which is positioned near and outside the blood vessel. The intravascular device is fixed at a predetermined position in a blood vessel to make it possible to stably measure brain waves. Furthermore, since the intravascular device is inserted into an extremely thin blood vessel, identification of a position of placement in the tissue is important in medical practices.

In the intravascular device, the electrode may be provided to a wire member, offering, in this case, a smaller dilation force, compared with that of a stent, superior ease of sliding with respect to a catheter, higher ease of bending, and superior ease of delivery into a brain blood vessel having a smaller diameter. Furthermore, the wire member is preferable in terms of that, since contact with a blood vessel is suppressed (in particular, a wire member having a bar shape in a natural state rarely comes into contact with a wall of a blood vessel), an adverse event rarely occurs even when placed for a long period of time. Therefore, it is preferable that it be placed in a blood vessel for a day or longer. The intravascular device includes a core body and an insulating body. More specifically, an outer periphery of the core body may be covered with the insulating body. As the core body, an extremely thin wire made of stainless-steel or nickel-titanium alloy may be used, for example, and an example of the extremely thin wire serving as the core body may be one having a diameter ranging from approximately 0.1 mm to approximately 1 mm, for example. An example of the insulating body may be a piece of polyimide tube or a piece of PTFE tube, for example. Note that, although the insulating body itself has a cylindrical body, it is internally filled with the core body, making it possible to regard the intravascular device as a wire member.

Furthermore, the intravascular device may include an intravascular electrode and an intravascular spare electrode, which are provided to an identical wire member but are slightly separated away from each other. Note that, the present invention is not limited to the examples, and a number of electrodes provided to one intravascular device may be one or plural such as three. By using such a thin intravascular device with no dilation force as described above, the intravascular device is easily delivered to a position near a first site even in a periphery portion of a blood vessel. In other words, since the intravascular device of this type offers a high degree of freedom in installation at a site in a blood vessel, it is not necessary to perform a delivery operation while a second site is understood. This feature is in contrast to that of a stent type intravascular device, whose intrinsic size and dilation force naturally constrain installation at a site in a blood vessel.

Note that the intravascular device may have a portion including an X-ray impermeable member (for example, one made of platinum). In this case, similar to a case when using an angio image AI (a fourth image) as described above, for example, it is possible to understand in a real-time manner a position of the intravascular device in a blood vessel. The server 1 acquires information of a potential acquired from the intravascular electrode (the intravascular device, via the terminal 2), and computes a result of measurement of brain waves (brain wave analysis).

The medical doctor DC identifies a site that should be cut (for example, a tumor) based on the result of measurement of brain waves. Then, the medical doctor DC performs a surgical operation of cutting an identified part of the brain tissue. The image display and the brain wave analysis will now be described herein in detail.

### <System Configuration>

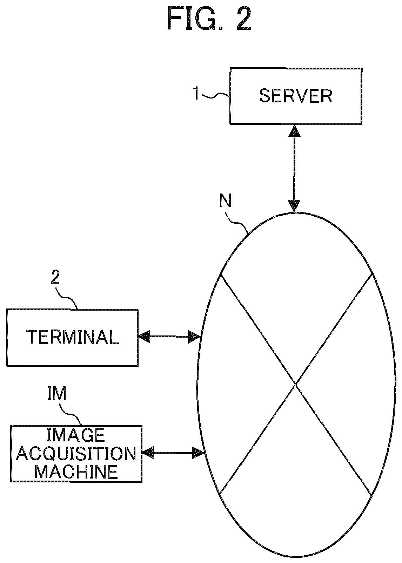

Fig. 2 is a diagram illustrating an outline of a configuration of a system according to the present embodiment. In the system according to the present embodiment, the server 1 that performs processing, the terminal 2, and the image acquisition machine IM are coupled to each other via a predetermined network N such as the Internet, for example. Furthermore, the terminal 2 and the intravascular device are coupled to each other.

The server 1 cooperates with the terminal 2 and the image acquisition machine IM in operation to execute various types of processing. The terminal 2 may display a clinical record of the patient PT.

### <Hardware Configuration>

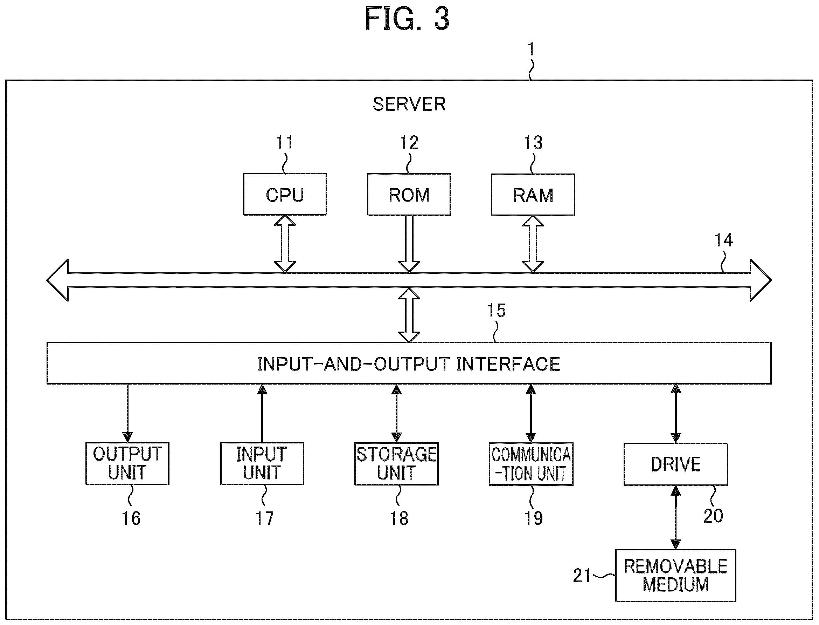

Fig. 3 is a block diagram illustrating a hardware configuration of the server 1 according to the present embodiment. The server 1 includes a central processing unit (CPU) 11, a read only memory (ROM) 12, a random access memory (RAM) 13, a bus 14, an input-and-output interface 15, an output unit 16, an input unit 17, a storage unit 18, a communication unit 19, and a drive 20.

The CPU 11 executes various types of processing in accordance with programs recorded in the ROM 12 or programs loaded from the storage unit 18 to the RAM 13. The RAM 13 appropriately stores, for example, data necessary for the CPU 11 when executing various types of processing. The CPU 11, the ROM 12, and the RAM 13 are coupled to each other via the bus 14. The bus 14 is further coupled to the input-and-output interface 15.

The input-and-output interface 15 is coupled to the output unit 16, the input unit 17, the storage unit 18, the communication unit 19, and the drive 20. The output unit 16 includes a display and a loud-speaker to output various types of information in the form of image and audio, for example. The input unit 17 includes a keyboard and a mouse to accept various types of information, for example. The storage unit 18 includes a hard disk and a dynamic random access memory (DRAM) to store various types of data, for example. The communication unit 19 performs communication with other machines via the network N including the Internet.

The drive 20 is appropriately attached with a removable medium 21 such as a magnetic disk, an optical disk, a magnetic optical disk, or a semiconductor memory. A program read from the removable medium 21 by the drive 20 is installed into the storage unit 18 as required. Furthermore, the removable medium 21 is able to store various types of data stored in the storage unit 18, similar to the storage unit 18.

Note that, although not illustrated, the terminal 2 has a hardware configuration illustrated in Fig. 3.

### <Functional Configuration>

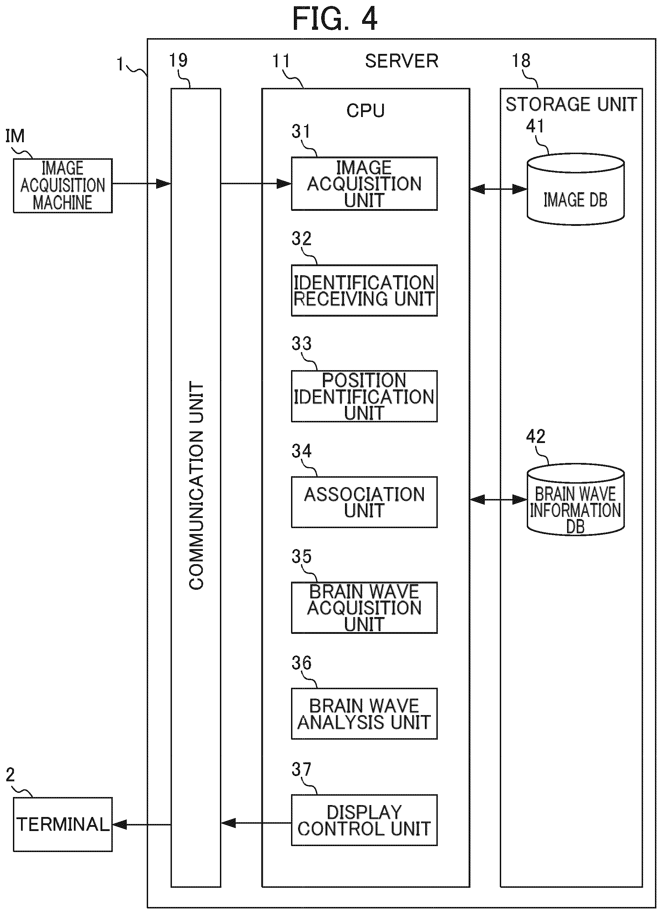

Fig. 4 is a functional block diagram illustrating an example of a functional configuration of the server 1 according to the present embodiment.

When the CPU 11 in the server 1 operates, an image acquisition unit 31, an identification receiving unit 32, a position identification unit 33, an association unit 34, a brain wave acquisition unit 35, a brain wave analysis unit 36, and a display control unit 37 function. Furthermore, the storage unit 18 in the server 1 is provided with an image database (DB) 41 and a brain wave information DB 42.

The image acquisition unit 31 acquires a brain tissue image BI and a blood vessel image VI pertaining to the patient PT, which are acquired by the image acquisition machine IM. Information of various types of images acquired by the image acquisition unit 31 is stored in the image DB 41.

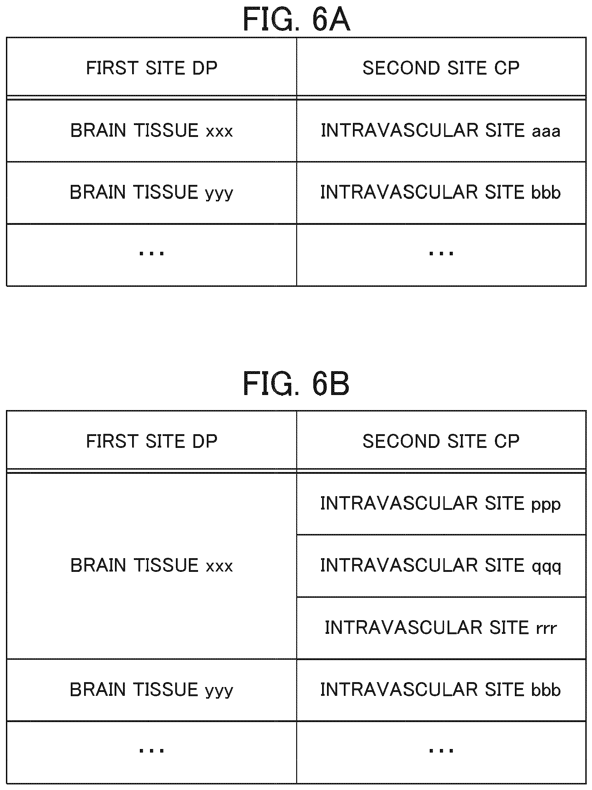

The identification receiving unit 32 (a receiving unit) receives, from the medical doctor DC, identification of a first site DP on the brain tissue image BI. In the present embodiment, the identification receiving unit 32 receives identification of a first site DP, which is provided by the medical doctor DC, in a state where the terminal 2 is displaying the brain tissue image BI. It is possible to regard the first site DP as a portion in the brain tissue (for example, a temporal lobe, a hippocampus, an amygdaloid nucleus, or a cortical node), which the medical doctor DC desires to measure brain waves. Note that a method for identifying a first site DP is not particularly limited, and, for example, the identification receiving unit 32 may receive, instead of an image, selection of a portion in the brain tissue from the medical doctor DC to receive identification of a first site DP. In this case, the storage unit 18 may be prepared beforehand with an association table (Fig. 6A or 6B) in which a position (or a portion or a region) in the brain tissue and a position (or a portion or a region) of a blood vessel are associated with each other, and the position identification unit 33 described later may refer to the association table to identify a second site CP described later.

The position identification unit 33 (an identification unit) identifies a second site CP corresponding to the first site DP described above on the blood vessel image VI. Note herein that a first site DP is a site in the brain tissue, and a second site CP is a site corresponding to the first site DP in a blood vessel (a stream). It is desirable that a second site CP be a site in a blood stream, which lies at a shortest distance (for example, an Euclidean distance) to the first site DP in the brain tissue. A reason of this is that it makes it possible to further accurately acquire, at the second site CP in the blood vessel, brain waves generated at the first site DP. Note that an identification method is not particularly limited, and it is possible to apply one of various types of methods. The position identification unit 33 uses the association table described above to identify a second site CP corresponding to a first site DP. Furthermore, when a brain tissue image BI and a blood vessel image VI are three-dimensional images, for example, the position identification unit 33 may perform matching in positional relationship between the brain tissue image BI and the blood vessel image VI based on their positions of centers of gravity and cross-sectional directions, respectively, to identify a second site CP corresponding to a first site DP described above. Furthermore, when a synthesized image FI has been manually synthesized by the medical doctor DC, for example, the synthesized image FI may be used to identify a second site CP corresponding to a first site DP described above.

The position identification unit 33 identifies a second site CP based on information about the intravascular device (the device). The information about the intravascular device includes information regarding a size of the intravascular device and optionally information on ease of delivery of the intravascular device into a blood vessel and information regarding a dilation force of the intravascular device. For example, when a wire is used, information about the intravascular device (wire information) includes its effective length and its diameter (when released) and a diameter of a catheter suitable for combined use (interpolation). For example, the position identification unit 33 acquires a position at which it is possible to dispose the intravascular device in accordance with the ease of delivery into a blood vessel, the size, and the dilation force of the intravascular device. The position identification unit 33 acquires a position or a region in a blood vessel (for example, a blood vessel that is greater in blood vessel diameter than a superior sagittal sinus), at which it is possible to dispose the intravascular device, in accordance with the size of the intravascular device. Then, the position identification unit 33 identifies, as a second site CP, a site in a blood stream in the blood vessel, which lies at a shortest distance to the first site DP, and at which it is possible to make the disposition. Note herein that the ease of delivery into a blood vessel is an index indicating ease of sliding with respect to a catheter. The higher the ease of sliding, the superior the ease of delivery into a blood vessel. Furthermore, the ease of expansion is an index indicating dilation force of a blood vessel. By causing an intravascular device provided with an electrode to expand in a brain blood vessel and to be put in place on a wall of the blood vessel to sense electrical activity in a nearby part of nerve tissue, it is necessary that, when the intravascular device being used has higher ease of expansion, for example, a diameter of a blood vessel, in which the intravascular device is to be put in place, must be greater (thicker) to some extent. Note that a method for acquiring information about an intravascular device is not particularly limited, and, for example, information about an intravascular device may be received by a non-illustrated receiving unit (a device receiving unit) or acquired from a DB stored in the storage unit 18 beforehand.

The association unit 34 associates the brain tissue image BI and the blood vessel image VI with each other. For example, when a brain tissue image BI and a blood vessel image VI are three-dimensional images, the association unit 34 may associate the brain tissue image BI and the blood vessel image VI with each other in positional relationship based on their positions of centers of gravity and cross-sectional directions, respectively. Note that the association unit 34 may associate a brain tissue image BI and a blood vessel image VI with each other to generate a synthesized image FI.

The brain wave acquisition unit 35 acquires brain waves from the intravascular device inserted into the brain blood vessel in the patient PT by the medical doctor DC. Specifically, the brain wave acquisition unit 35 acquires information about a potential acquired from the intravascular electrode in the intravascular device. The acquired brain waves are stored in the brain wave information DB 42.

The brain wave analysis unit 36 analyzes the brain waves acquired by the brain wave acquisition unit 35. For example, the brain wave analysis unit 36 applies Fourier transformation on the brain waves described above to generate information for use by the medical doctor DC for performing diagnosis.

The display control unit 37 (a display control unit) causes the terminal 2 to display various types of information. For example, the display control unit 37 causes the terminal 2 to display a brain tissue image BI and a blood vessel image VI. Then, the display control unit 37 causes the terminal 2 to display items indicating a first site DP on the brain tissue image BI and a second site CP on the blood vessel image VI. Note herein that the items are icons such as dots and frames indicating the first site DP and the second site CP. Note that the display control unit 37 may cause the terminal 2 to not display a brain tissue image BI (a first image), but to display a blood vessel image VI (a second image) and an item indicating a second site CP. Furthermore, for example, the display control unit 37 causes the terminal 2 to display, as the brain wave analysis unit 36 has performed analysis, a result of the analysis.

### <Content of Processing: Image Display Processing>

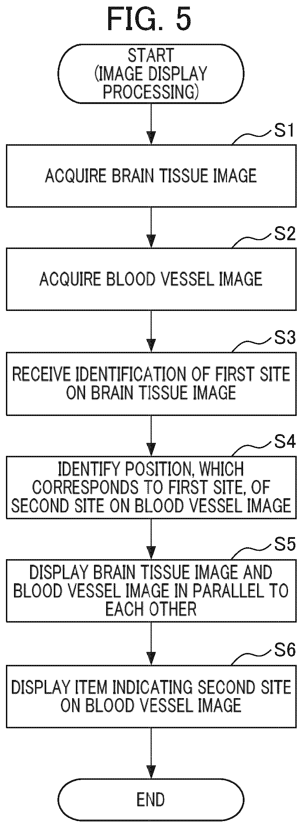

Fig. 5 is a diagram illustrating an example of image display processing according to the present embodiment. In Fig. 5, the server 1 acquires, from the image acquisition machine IM, and causes the terminal 2 to display a brain tissue image BI and a blood vessel image VI. Fig. 5 illustrates, for example, the image display processing when the position identification mode is ON.

In step S1, the image acquisition unit 31 acquires and stores, in the image DB 41, a brain tissue image BI acquired by the image acquisition machine IM. The brain tissue image BI is, for example, an image of the brain tissue, which is acquired by using MRI.

In step S2, the image acquisition unit 31 acquires and stores, in the image DB 41, a blood vessel image VI acquired by the image acquisition machine IM. The blood vessel image VI is, for example, a magnetic resonance image (MRA) of the blood vessels, which is acquired by using the MRI described above.

In step S3, the identification receiving unit 32 receives identification of a first site DP on the brain tissue image BI. For example, the identification receiving unit 32 receives identification of a first site DP as the medical doctor DC selects a part on the brain tissue image BI or selects a partial portion in the brain tissue.

In step S4, the position identification unit 33 identifies, as described above, a position of a second site CP on the blood vessel image VI, which corresponds to the first site DP on the brain tissue image BI, which is identified in step S4.

The According to an example not covered by the claimed invention, position identification unit 33 uses the association table illustrated in Fig. 6A to identify a second site CP, for example. The association table illustrated in Fig. 6A is stored beforehand in the storage unit 18, for example. Fig. 6A illustrates an example of the association table according to the present example . In the association table illustrated in Fig. 6A, a first site in the brain tissue and a second site in a blood vessel, which corresponds to the first site, are associated with each other. For example, when the identification receiving unit 32 has received "brain tissue xxx" that is a part of the brain tissue as a first site DP, the position identification unit 33 refers to that illustrated in Fig. 6A to identify an "intravascular site aaa" that is a site in a blood vessel, which corresponds to the "brain tissue xxx", as a second site CP. According to the invention, when information about the intravascular device has been acquired, as described above, the position identification unit 33 uses the association table illustrated in Fig. 6B to identify a second site CP, for example. The association table illustrated in Fig. 6B is stored beforehand in the storage unit 18, for example. Fig. 6B illustrates an embodiment of the association table according to the present invention. In the association table illustrated in Fig. 6B, a first site in the brain tissue and one or more second sites in blood vessels, which correspond to the first site, are associated with each other. For example, when the identification receiving unit 32 has received "brain tissue xxx" that is a part of the brain tissue as a first site DP and has acquired information about the intravascular device (for example, a size and ease of expansion), the position identification unit 33 refers to that illustrated in Fig. 6B to acquire an "intravascular site ppp", an "intravascular site qqq", and an "intravascular site rrr" that are sites in blood vessels, which correspond to the "brain tissue xxx". Then, the position identification unit 33 identifies a site at which it is possible to dispose the intravascular device among the "intravascular site ppp", the "intravascular site qqq", and the "intravascular site rrr". At this time, it is desirable that information about a diameter of a blood vessel and a distance from a corresponding part of brain tissue be associated with an intravascular site and recorded in the association table. Thereby, the position identification unit 33 is able to take into account a diameter of a blood vessel and a size of the intravascular device to identify, among sites at which it is possible tc dispose the intravascular device, a site that is closest in distance to a corresponding part of tissue, as a second site CP. Note that it is not limited that the position identification unit 33 uses one of the tables described above, but may use, according to other examples not covered by the claimed invention, a predetermined mathematical function that outputs a second site CP when a first site DP and information about an intravascular device (a size and ease of expansion, for example) are inputted to identify a second site CP.

In step S5, the display control unit 37 causes the terminal 2 to display the brain tissue image BI and the blood vessel image VI in parallel to each other. It is to be noted that, in the present embodiment, providing parallel display includes displaying a brain tissue image BI and a blood vessel image VI on a single display unit in a non-overlapped manner or on a plurality of display units in a separated manner and displaying a brain tissue image BI and a blood vessel image VI on a display unit in a partially or fully overlapped manner. An example of displaying a brain tissue image BI and a blood vessel image VI in a partially overlapped manner may be displaying the blood vessel image VI that has been shrunk at an end of the brain tissue image BI. An example of displaying a brain tissue image BI and a blood vessel image VI in a fully overlapped manner may be adjusting a degree of permeability of one or both of the images to display the images in an overlapped manner. Furthermore, when a brain tissue image BI and a blood vessel image VI are displayed in parallel to each other, and a display angle or a display mode is changed (to a cross-sectional 2D display mode or a 3D display mode, for example) for one of the images, the display control unit 37 may change a display angle or a display mode for the other one of the images in accordance with (in line with) the change. Note that the display control unit 37 may cause the display unit of the terminal 2 to display a synthesized image described above.

In step S6, the display control unit 37 causes an item indicating the second site CP to be displayed on the blood vessel image VI. Note that the display control unit 37 may cause an item indicating the first site DP to be displayed on the brain tissue image BI.

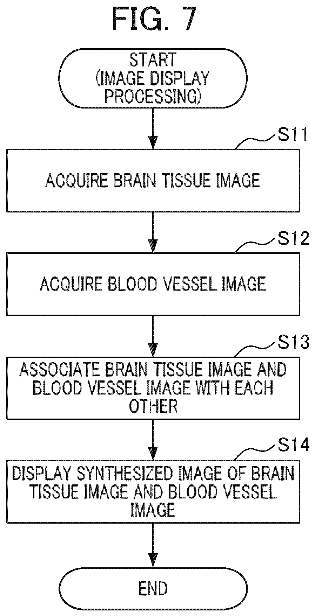

Fig. 7 is a diagram illustrating another example of the image display processing according to the present embodiment. In Fig. 6, the server 1 acquires, from the image acquisition machine IM, and causes the terminal 2 to display a brain tissue image BI and a blood vessel image VI. Fig. 6 illustrates, for example, the image display processing when the position identification mode is OFF.

Since steps S11 and S12 are similar or identical to steps S1 and S2 described above, their descriptions are omitted.

In step S13, the association unit 34 associates the brain tissue image BI and the blood vessel image VI with each other, as described above. Furthermore, the association unit 34 generates a synthesized image FI in which the brain tissue image BI and the blood vessel image VI, which are associated with each other, are synthesized with each other. As described above, when a brain tissue image BI and a blood vessel image VI are three-dimensional images, for example, the association unit 34 may associate the brain tissue image BI and the blood vessel image VI with each other in positional relationship based on their positions of centers of gravity and cross-sectional directions, respectively. Note that the association unit 34 may acquire a synthesized image FI in which a brain tissue image BI and a blood vessel image VI are manually synthesized with each other by a person such as the medical doctor DC. In a method for generating a synthesized image FI, for example, the medical doctor DC acquires a piece of data of the brain tissue and a piece of data of blood vessels, which are acquired through MRI. Then, the medical doctor DC uses predetermined software, and causes the piece of data of the brain tissue and the piece of data of the blood vessels to be overlapped with each other to generate a synthesized image FI. Note that, when a machine for MRI synthesizes a brain tissue image BI and a blood vessel image VI with each other, for example, the association unit 34 may acquire a synthesized image FI that has been synthesized through MRI.

In step S14, the display control unit 37 causes the terminal 2 to display the synthesized image FI in which the brain tissue image BI and the blood vessel image VI have been synthesized with each other.

### <Content of Processing: Brain Wave Analysis Processing>

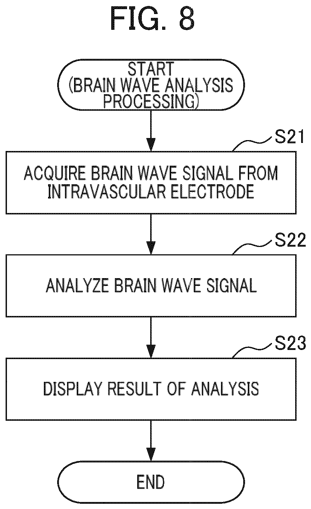

Fig. 8 is a diagram illustrating an example of the image display processing according to the present embodiment. In Fig. 8, the server 1 acquires brain waves from the intravascular device, analyzes the brain waves, and causes the terminal 2 to display a result of the analysis. Specifically, in step S21, the brain wave acquisition unit 35 acquires, from the intravascular electrode, and stores, in the brain wave information DB 42, brain waves (a brain wave signal). Then, in step S22, the brain wave analysis unit 36 analyzes the acquired brain waves. Furthermore, in step S23, the display control unit 37 causes the terminal 2 to display a result of the analysis.

### <Blood Streams in Blood Vessels in Brain>

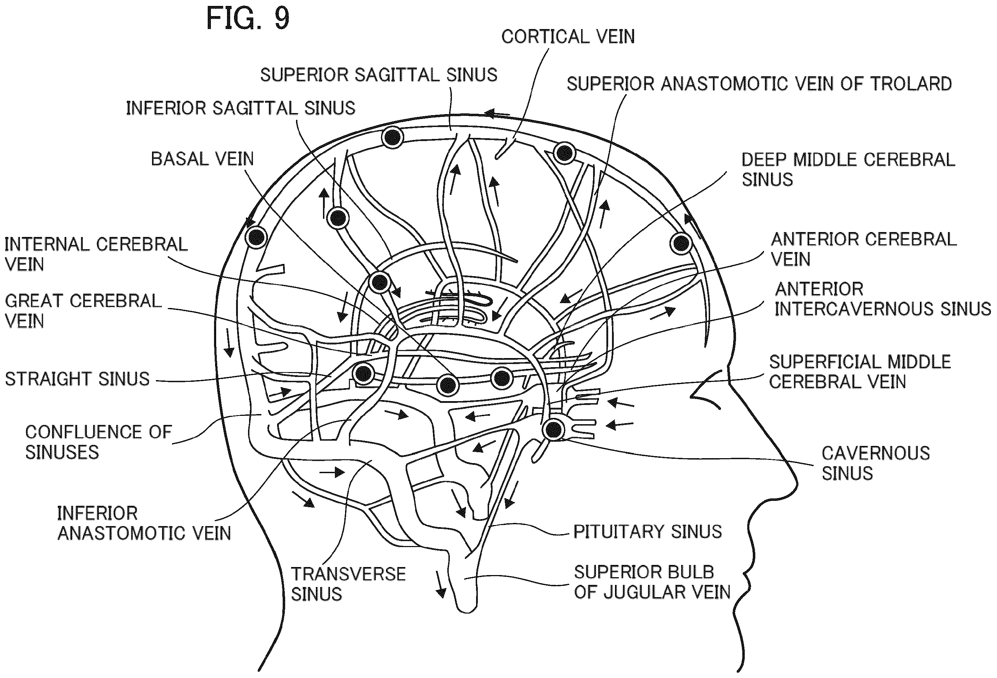

Fig. 9 is a diagram illustrating an example of blood streams in blood vessels in a brain. For example, using a thin intravascular device having a diameter of approximately 0.25 millimeters makes it possible to place the intravascular electrode in a peripheral portion (a thin portion) of a blood vessel. For example, it is assumed that such an intravascular electrode as described above be placed at one of sites (regions) indicated by black circles in Fig. 9. For example, a second site CP may be a position in a blood vessel on an upstream side of a superior sagittal sinus. Note herein that a blood vessel on the upstream side of the superior sagittal sinus refers to a blood vessel thinner than the superior sagittal sinus and a blood vessel lying at a position on an upstream side in blood flow. Furthermore, for example, a second site CP may be a position in a blood vessel when the blood vessel has an inner diameter equal to or greater than 1 mm and equal to or smaller than 10 mm. The inner diameter of a blood vessel may be a diameter of a circular blood vessel or an inner diameter at a largest site in an oval blood vessel. Note that the values 1 mm and 10 mm are mere examples, and there may be a position in a blood vessel when the blood vessel has an inner diameter falling within a predetermined range. Furthermore, a second site CP may be a position in a blood vessel within a predetermined region (for example, one of regions indicated by black circles in Fig. 9).

### <Advantageous Effects of the Present Embodiment>

According to the present embodiment described above, for example, it is possible to use proper disposition of the intravascular devices according to the present invention at sites in brain blood vessels near a left brain and a right brain, respectively, to detect brain waves for identifying a focal point of epilepsy and for detecting a seizure of epilepsy. Associating the brain tissue and a blood vessel with each other to indicate a second site in the blood vessel, which corresponds to a first site in the brain tissue, makes it possible to allow a medical doctor to easily understand a site at which the intravascular electrode is to be disposed to properly sense electrical activity in biological tissue that generates an electric signal. Furthermore, according to the present embodiment described above, displaying a synthesized image of a brain tissue image and a blood vessel image makes it possible to allow a medical doctor to easily understand a site at which the intravascular electrode is to be disposed to properly sense electrical activity in biological tissue that generates an electric signal. Thereby, it is possible to achieve higher spatial resolution and higher time resolution in measuring brain waves at a first site in the brain tissue. Furthermore and thereby, since it is possible to place the intravascular electrode in a blood vessel for a long period of time, compared with a case when placing an intracranial electrode, it is possible to continuously measure brain waves. Furthermore and thereby, since a medical doctor who is able to perform intravascular medical operations is at least required, instead of a special medical doctor for neurosurgical operations, it is possible to increase a number of facilities in which it is possible to implement such operations, compared with a case when applying a subdural electrode or inserting an electrode for depth electroencephalogram.

Furthermore, according to the present embodiment described above, performing the analysis processing on brain waves acquired from the intravascular electrode makes it possible to allow a state of the brain waves to be understood in a real-time manner.

Furthermore, according to the present embodiment described above, by indicating a thinner portion of a blood vessel as a second site, the intravascular electrode can be implanted even in a peripheral portion that is difficult to reach with a conventional intravascular device but can be reached, for example, by using a thinner (approximately 0.25 millimeters) intravascular device with a smaller dilation force, thereby making it possible to perform various types of diagnoses. Since such an intravascular device as described above is extremely minimally invasive, compared with an intracranial electrode, there are less side effects and less risk of disease complications even when the intravascular device is used. Furthermore, although an intracranial electrode only measures a potential on a brain surface, the intravascular device described above is able to acquire a potential deep inside the brain, making it possible to monitor the brain region in a wider range. Furthermore, an intracranial electrode requires a craniotomy, limiting an area onto which application is possible. On the other hand, the intravascular devices described above are able to be easily placed even at portions separated away at a greater distance, such as the forehead and the back of the head.

Although the embodiment of the present invention has been described, the present invention is not limited to the embodiment described above. The present invention still includes amendments and modifications, for example, that fall within the scope of the claims.

### (Modification Example)

Although, in the present embodiment described above, described is an example where an image of the brain tissue and a blood vessel image are displayed in an examination for a surgical operation for refractory epilepsy, an image of the brain tissue and a blood vessel image may be displayed in a similar manner to those described above, as long as its purpose is for diagnosis of epilepsy.

Although, in the present embodiment described above, an example has been described where an image of the brain tissue and a blood vessel image are displayed in parallel to each other or a synthesized image is displayed in accordance with a mode (the position identification mode), the present invention is not limited to the example. For example, when one or more events such as when an intravascular device has approached, reached, and passed a second site are detected based on information indicating a second site and information of an angio image, the detection may be notified (displayed) in a real-time manner. Furthermore, for example, no mode may be provided, but an image of the brain tissue and a blood vessel image may be displayed. Furthermore, for example, no mode may be provided, but a synthesized image may be displayed. Furthermore, for example, an image of the brain tissue, a blood vessel image, and a synthesized image may be displayed in parallel to each other.

Although, in the present embodiment described above, described is an example where MRI is used to acquire an image of the brain tissue, the present invention is not limited to the example. An image of the brain tissue may be acquired by using computed tomography (CT) or magneto encephalography (MEG) .

Although, in the present embodiment described above, MRA has been used to acquire a blood vessel image, a method for acquiring a blood vessel image is not particularly limited, and such an image may be acquired by using one of various types of methods.

Although, in the present embodiment described above, described is an example where the intravascular electrode senses an electric signal generated in biological tissue (measures brain waves), the present invention is not limited to the example, and the intravascular electrode may be used for various types of purposes.

Furthermore, for example, it is possible to use hardware or software to execute the series of processing described above. In other words, the functional configuration described above is a mere example, and the present invention is not particularly limited to the functional configuration. That is, it is enough that the system described above has functions that make it possible to wholly execute the series of processing described above, and functional blocks used to realize the functions are not particularly limited to the functional blocks illustrated in the examples described above. Furthermore, locations at which the functional blocks are present are not particularly limited to the locations illustrated in Fig. 4, and desired locations may be selected. For example, the functional blocks in the server may be transferred to another machine. Contrarily, a functional block in another machine may be transferred to the server, for example. Furthermore, a single piece of hardware may configure one functional block. A single piece of software may configure one functional block. A combination of pieces of hardware and software may configure one functional block.

To execute, with software, the series of processing, a program configuring the software is installed into a computer from a network or a recording medium, for example. The computer may be such a computer incorporated in special hardware. Furthermore, the computer may be such a computer installed with various programs used to execute various functions, such as, in addition to servers, general-purpose smart phones and personal computers.

A recording medium storing such programs as described above may not only be a non-illustrated removable medium distributed separately from a machine main body to provide the programs to each user, for example, but also be a recording medium provided to each user, for example, in a state where the recording medium is assembled beforehand in the machine main body, for example.

Note that, in the present specification, steps describing programs recorded in a recording medium include not only processes sequentially executed in a chronological order, but also processes that may not necessarily be executed in a chronological order, but may be executed in parallel or separately. Furthermore, in the present specification, the term system means a generic apparatus including a plurality of machines and a plurality of unit, for example.

### EXPLANATION OF REFERENCE NUMERALS

1: Server
2: Terminal
11: CPU
18: Storage unit
19: Communication unit
31: Image acquisition unit
32: Identification receiving unit
33: Position identification unit
34: Association unit
35: Brain wave acquisition unit
36: Brain wave analysis unit
37: Display control unit

## Claims

1. An information processing machine for diagnosis of epilepsy, comprising:
an image acquisition unit that acquires a brain tissue image (BI) and a blood vessel image (VI) of a subject from an image acquisition machine (IM);
a receiving unit (32) that receives identification of a first site (DP) that is in the brain tissue image (BI) and is a target from which an electric signal is to be detected;
a wire information receiving unit and a device receiving unit that receives wire information, wherein the wire information includes a size of an epilepsy diagnosis device to be disposed in a blood vessel;
an identification unit (33) that is configured to identify, based on the wire information, a second site (CP) that is in the blood vessel image (VI) and corresponds to the first site (DP);
wherein the identification unit (33) identifies, among blood vessels in or near the brain tissue in the subject, the second site (CP) that is suitable for the epilepsy diagnosis device to be disposed to detect an electric signal from the first site (DP) based on the wire information; and
wherein, with reference to an association table which is stored beforehand in a storage unit and in which the first site (DP) and one or more of sites in the blood vessels, which correspond to the first site (DP), are associated with each other, and under taking into account a blood vessel diameter and the size of the epilepsy diagnosis device, the identification unit (33) identifies, among the one or more sites at which it is possible to dispose the epilepsy diagnosis device, a site that is closest in distance to the first site (DP) as the second site (CP); and
a display control unit (37) that causes a predetermined display unit to display an item indicating the second site (CP) in the blood vessel image (VI).

2. The information processing machine, according to claim 1, wherein the wire information includes information regarding ease of delivery of the epilepsy diagnosis device into a blood vessel.

3. The information processing machine, according to claim 1 or 2, wherein the wire information further includes information regarding a dilation force of the epilepsy diagnosis device.

4. The information processing machine, according to any one of claims 1 to 3, wherein the second site (CP) is a position in the blood vessel on an upstream side in blood flow of a superior sagittal sinus.

5. The information processing machine, according to any one of claims 1 to 4, wherein the second site (CP) is a position in the blood vessel, the position in the blood vessel having an inner diameter equal to or greater than 1 mm and equal to or smaller than 10 mm.

6. The information processing machine, according to any one of claims 1 to 5, wherein the display control unit (37) causes the display unit to display the brain tissue image (BI) and the blood vessel image (VI), in parallel to each other.

7. The information processing machine according to claim 6, wherein the display control unit (37) further causes a predetermined display unit to display a fourth image (AI) acquired through a blood vessel contrast-imaging examination, in addition to the brain tissue image (BI) and the blood vessel image (VI), in parallel to each other.

8. A computer implemented information processing method for diagnosis of epilepsy, comprising:
an image acquisition step of acquiring a brain tissue image (BI) and a blood vessel image (VI) of a subject from an image acquisition machine (IM);
a receiving step of receiving identification of a first site (DP) that is in the brain tissue image (BI) and is a target from which an electric signal is to be detected;
a wire information receiving step and a device receiving step of receiving wire information, wherein the wire information includes a size of an epilepsy diagnosis device to be disposed in a blood vessel;
an identification step of identifying, based on wire information, a second site (CP) that is in the blood vessel image (VI) and corresponds to the first site (DP),
wherein the identification step comprises identifying, among blood vessels in or near the brain tissue in the subject, the second site (CP) that is suitable for the epilepsy diagnosis device to be disposed to detect an electric signal from the first site (DP), based on the wire information, and
wherein with reference to an association table which is stored beforehand in a storage unit and in which the first site (DP) and one or more sites in the blood vessels, which correspond to the first site (DP), are associated with each other, and under taking into account a blood vessel diameter and the size of the epilepsy diagnosis device, the identification unit (33) identifies, among the one or more sites at which it is possible to dispose the epilepsy diagnosis device, a site that is closest in distance to the first site (DP) as the second site (CP); and
a display control step that causes a predetermined display unit to display an item indicating the second site (CP) in the blood vessel image (VI).

9. A computer program for diagnosis of epilepsy, causing a computer to execute:
an image acquisition step of acquiring a brain tissue image (BI) and a blood vessel image (VI) of a subject from an image acquisition machine (IM);
a receiving step of receiving identification of a first site (DP) that is in the brain tissue image (BI) and is a target from which an electric signal is to be detected;
a wire information receiving step and a device receiving step of receiving wire information, wherein the wire information includes a size of an epilepsy diagnosis device to be disposed in a blood vessel;
an identification step of identifying, based on wire information, a second site (CP) that is in the blood vessel image (VI) and corresponds to the first site (DP),
wherein the identification step comprises identifying, among blood vessels in or near the brain tissue in the subject, the second site (CP) that is suitable for the epilepsy diagnosis device to be disposed to detect an electric signal from the first site (DP), based on wire information, and
wherein, with reference to an association table which is stored beforehand in a storage unit and in which the first site (DP) and one or more sites in the blood vessels, which correspond to the first site (DP), are associated with each other, and under taking into account a blood vessel diameter and the size of the epilepsy diagnosis device, the identification unit (33) identifies, among the one or more sites at which it is possible to dispose the epilepsy diagnosis device, a site that is closest in distance to the first site (DP) as the second site (CP); and
a display control step that causes a predetermined display unit to display an item indicating the second site (CP) in the blood vessel image (VI).

## Patentansprüche

1. Informationsverarbeitungsmaschine zur Diagnose von Epilepsie, aufweisend:
eine Bilderfassungseinheit, die ein Hirngewebebild (BI) und ein Blutgefäßbild (VI) eines Subjekts von einer Bilderfassungsmaschine (IM) erfasst;
eine Empfangseinheit (32), die eine Identifizierung einer ersten Stelle (DP) empfängt, die sich in dem Hirngewebebild (BI) befindet und ein Ziel ist, von dem ein elektrisches Signal erfasst werden soll;
eine Drahtinformations-Empfangseinheit und eine Vorrichtungs-Empfangseinheit, die Drahtinformation empfängt, wobei die Drahtinformation eine Größe einer Epilepsiediagnosevorrichtung mit einschließt, die in einem Blutgefäß anzuordnen ist;
eine Identifizierungseinheit (33), die dazu ausgebildet ist, basierend auf der Drahtinformation eine zweite Stelle (CP) zu identifizieren, die sich in dem Blutgefäßbild (VI) befindet und der ersten Stelle (DP) entspricht;
wobei die Identifizierungseinheit (33) unter Blutgefäßen in oder nahe dem Hirngewebe in dem Subjekt, basierend auf der Drahtinformation, die zweite Stelle (CP) identifiziert, die dafür geeignet ist, die Epilepsiediagnosevorrichtung dort anzuordnen, um ein elektrisches Signal von der ersten Stelle (DP) zu erfassen; und
wobei, unter Bezugnahme auf eine Zuordnungstabelle, die zuvor in einer Speichereinheit gespeichert ist und in der die erste Stelle (DP) und eine oder mehrere Stellen in den Blutgefäßen, die der ersten Stelle (DP) entsprechen, miteinander assoziiert sind, und unter Berücksichtigung eines Blutgefäßdurchmessers und der Größe der Epilepsiediagnosevorrichtung, die Identifizierungseinheit (33) unter der einen oder den mehreren Stellen, an denen es möglich ist, die Epilepsiediagnosevorrichtung anzuordnen, eine Stelle, die der ersten Stelle (DP) hinsichtlich der Entfernung am nächsten liegt, als die zweite Stelle (CP) identifiziert; und
eine Anzeigesteuereinheit (37), die bewirkt, dass eine vorbestimmte Anzeigeeinheit ein Element anzeigt, das die zweite Stelle (CP) in dem Blutgefäßbild (VI) angibt.

2. Informationsverarbeitungsmaschine nach Anspruch 1, wobei die Drahtinformation eine Information bezüglich des Einführkomforts der Epilepsiediagnosevorrichtung in ein Blutgefäß mit einschließt.

3. Informationsverarbeitungsmaschine nach Anspruch 1 oder 2, wobei die Drahtinformation ferner eine Information bezüglich einer Aufweitungskraft der Epilepsiediagnosevorrichtung mit einschließt.

4. Informationsverarbeitungsmaschine nach einem der Ansprüche 1 bis 3, wobei die zweite Stelle (CP) eine Position in dem Blutgefäß auf einer in Blutflussrichtung stromaufwärtigen Seite eines Sinus sagittalis superior ist.

5. Informationsverarbeitungsmaschine nach einem der Ansprüche 1 bis 4, wobei die zweite Stelle (CP) eine Position in dem Blutgefäß ist, wobei die Position in dem Blutgefäß einen Innendurchmesser von gleich oder größer als 1 mm und gleich oder kleiner als 10 mm aufweist.

6. Informationsverarbeitungsmaschine nach einem der Ansprüche 1 bis 5, wobei die Anzeigesteuereinheit (37) bewirkt, dass die Anzeigeeinheit das Hirngewebebild (BI) und das Blutgefäßbild (VI) parallel zueinander anzeigt.

7. Informationsverarbeitungsmaschine nach Anspruch 6, wobei die Anzeigesteuereinheit (37) ferner bewirkt, dass eine vorbestimmte Anzeigeeinheit zusätzlich zu dem Hirngewebebild (BI) und dem Blutgefäßbild (VI) ein viertes Bild (AI), das durch eine Blutgefäß-Kontrastbildgebungsuntersuchung erfasst wurde, parallel zu diesen anzeigt.

8. Computerimplementiertes Informationsverarbeitungsverfahren zur Diagnose von Epilepsie, aufweisend:
einen Bilderfassungsschritt des Erfassens eines Hirngewebebildes (BI) und eines Blutgefäßbildes (VI) eines Subjekts von einer Bilderfassungsmaschine (IM);
einen Empfangsschritt des Empfangens einer Identifizierung einer ersten Stelle (DP), die sich in dem Hirngewebebild (BI) befindet und ein Ziel ist, von dem ein elektrisches Signal erfasst werden soll;
einen Drahtinformations-Empfangsschritt und einen Vorrichtungs-Empfangsschritt des Empfangens von Drahtinformation, wobei die Drahtinformation eine Größe einer Epilepsiediagnosevorrichtung mit einschließt, die in einem Blutgefäß anzuordnen ist;
einen Identifizierungsschritt des Identifizierens, basierend auf Drahtinformation, einer zweiten Stelle (CP), die sich in dem Blutgefäßbild (VI) befindet und der ersten Stelle (DP) entspricht,
wobei der Identifizierungsschritt aufweist, unter Blutgefäßen in oder nahe dem Hirngewebe in dem Subjekt, basierend auf der Drahtinformation, die zweite Stelle (CP) zu identifizieren, die dafür geeignet ist, die Epilepsiediagnosevorrichtung dort anzuordnen, um ein elektrisches Signal von der ersten Stelle (DP) zu erfassen, und
wobei, unter Bezugnahme auf eine Zuordnungstabelle, die zuvor in einer Speichereinheit gespeichert ist und in der die erste Stelle (DP) und eine oder mehrere Stellen in den Blutgefäßen, die der ersten Stelle (DP) entsprechen, miteinander assoziiert sind, und unter Berücksichtigung eines Blutgefäßdurchmessers und der Größe der Epilepsiediagnosevorrichtung, die Identifizierungseinheit (33) unter der einen oder den mehreren Stellen, an denen es möglich ist, die Epilepsiediagnosevorrichtung anzuordnen, eine Stelle, die der ersten Stelle (DP) hinsichtlich der Entfernung am nächsten liegt, als die zweite Stelle (CP) identifiziert; und
einen Anzeigesteuerschritt, der bewirkt, dass eine vorbestimmte Anzeigeeinheit ein Element anzeigt, das die zweite Stelle (CP) in dem Blutgefäßbild (VI) angibt.

9. Computerprogramm zur Diagnose von Epilepsie, das einen Computer veranlasst, Folgendes auszuführen:
einen Bilderfassungsschritt des Erfassens eines Hirngewebebildes (BI) und eines Blutgefäßbildes (VI) eines Subjekts von einer Bilderfassungsmaschine (IM);
einen Empfangsschritt des Empfangens einer Identifizierung einer ersten Stelle (DP), die sich in dem Hirngewebebild (BI) befindet und ein Ziel ist, von dem ein elektrisches Signal erfasst werden soll;
einen Drahtinformations-Empfangsschritt und einen Vorrichtungs-Empfangsschritt des Empfangens von Drahtinformation, wobei die Drahtinformation eine Größe einer Epilepsiediagnosevorrichtung mit einschließt, die in einem Blutgefäß anzuordnen ist;
einen Identifizierungsschritt des Identifizierens, basierend auf Drahtinformation, einer zweiten Stelle (CP), die sich in dem Blutgefäßbild (VI) befindet und der ersten Stelle (DP) entspricht,
wobei der Identifizierungsschritt aufweist, unter Blutgefäßen in oder nahe dem Hirngewebe in dem Subjekt, basierend auf Drahtinformation, die zweite Stelle (CP) zu identifizieren, die dafür geeignet ist, die Epilepsiediagnosevorrichtung dort anzuordnen, um ein elektrisches Signal von der ersten Stelle (DP) zu erfassen, und
wobei, unter Bezugnahme auf eine Zuordnungstabelle, die zuvor in einer Speichereinheit gespeichert ist und in der die erste Stelle (DP) und eine oder mehrere Stellen in den Blutgefäßen, die der ersten Stelle (DP) entsprechen, miteinander assoziiert sind, und unter Berücksichtigung eines Blutgefäßdurchmessers und der Größe der Epilepsiediagnosevorrichtung, die Identifizierungseinheit (33) unter der einen oder den mehreren Stellen, an denen es möglich ist, die Epilepsiediagnosevorrichtung anzuordnen, eine Stelle, die der ersten Stelle (DP) hinsichtlich der Entfernung am nächsten liegt, als die zweite Stelle (CP) identifiziert; und
einen Anzeigesteuerschritt, der bewirkt, dass eine vorbestimmte Anzeigeeinheit ein Element anzeigt, das die zweite Stelle (CP) in dem Blutgefäßbild (VI) angibt.

## Revendications

1. Machine de traitement d'informations pour le diagnostic de l'épilepsie, comprenant :
une unité d'acquisition d'images qui acquiert une image de tissu cérébral (BI) et une image de vaisseaux sanguins (VI) d'un sujet à partir d'une machine d'acquisition d'images (IM) ;
une unité de réception (32) qui reçoit l'identification d'un premier site (DP) qui se trouve dans l'image de tissu cérébral (BI) et est une cible à partir de laquelle un signal électrique doit être détecté ;
une unité de réception d'informations de fil et une unité de réception de dispositif qui reçoit des informations de fil, les informations de fil incluant une taille d'un dispositif de diagnostic d'épilepsie à disposer dans un vaisseau sanguin ;
une unité d'identification (33) qui est configurée pour identifier, sur la base des informations de fil, un deuxième site (CP) qui se trouve dans l'image de vaisseaux sanguins (VI) et correspond au premier site (DP) ;
dans lequel l'unité d'identification (33) identifie, parmi des vaisseaux sanguins dans le tissu cérébral ou à proximité de celui-ci dans le sujet, le deuxième site (CP) qui est approprié pour que le dispositif de diagnostic d'épilepsie soit disposé pour détecter un signal électrique à partir du premier site (DP) sur la base des informations de fil ; et
dans lequel, en référence à un tableau d'association qui est préalablement stocké dans une unité de stockage et dans lequel le premier site (DP) et un ou plusieurs sites dans les vaisseaux sanguins, qui correspondent au premier site (DP), sont associés l'un à l'autre, et en tenant compte d'un diamètre de vaisseau sanguin et de la taille du dispositif de diagnostic d'épilepsie, l'unité d'identification (33) identifie, parmi les un ou plusieurs sites où il est possible de disposer le dispositif de diagnostic d'épilepsie, un site qui est le plus proche en distance du premier site (DP) en tant que deuxième site (CP) ; et
une unité de commande d'affichage (37) qui amène une unité d'affichage prédéterminée à afficher un élément indiquant le deuxième site (CP) dans l'image de vaisseaux sanguins (VI).

2. Machine de traitement d'informations selon la revendication 1, dans laquelle les informations de fil incluent des informations concernant la facilité de distribution du dispositif de diagnostic d'épilepsie dans un vaisseau sanguin.

3. Machine de traitement d'informations, selon la revendication 1 ou 2, dans laquelle les informations de fil incluent en outre des informations concernant une force de dilatation du dispositif de diagnostic d'épilepsie.

4. Machine de traitement d'informations, selon l'une quelconque des revendications 1 à 3, dans laquelle le deuxième site (CP) est une position dans le vaisseau sanguin sur un côté amont dans le flux sanguin d'un sinus sagittal supérieur.

5. Machine de traitement d'informations, selon l'une quelconque des revendications 1 à 4, dans laquelle le deuxième site (CP) est une position dans le vaisseau sanguin, la position dans le vaisseau sanguin ayant un diamètre interne égal ou supérieur à 1 mm et égal ou inférieur à 10 mm.

6. Machine de traitement d'informations, selon l'une quelconque des revendications 1 à 5, dans laquelle l'unité de commande d'affichage (37) amène l'unité d'affichage à afficher l'image de tissu cérébral (BI) et l'image de vaisseaux sanguins (VI), parallèlement l'une à l'autre.

7. Machine de traitement d'informations selon la revendication 6, dans laquelle l'unité de commande d'affichage (37) amène en outre une unité d'affichage prédéterminée à afficher une quatrième image (AI) acquise par un examen d'imagerie par contraste de vaisseaux sanguins, en plus de l'image de tissu cérébral (BI) et de l'image de vaisseaux sanguins (VI), parallèlement les unes aux autres.

8. Procédé de traitement d'informations mis en œuvre par ordinateur pour le diagnostic de l'épilepsie, comprenant :
une étape d'acquisition d'images consistant à acquérir une image de tissu cérébral (BI) et une image de vaisseaux sanguins (VI) d'un sujet à partir d'une machine d'acquisition d'images (IM) ;
une étape de réception consistant à recevoir l'identification d'un premier site (DP) qui se trouve dans l'image de tissu cérébral (BI) et est une cible à partir de laquelle un signal électrique doit être détecté ;
une étape de réception d'informations de fil et une étape de réception de dispositif consistant à recevoir des informations de fil, les informations de fil incluant une taille d'un dispositif de diagnostic d'épilepsie à disposer dans un vaisseau sanguin ;
une étape d'identification consistant à identifier, sur la base des informations de fil, un deuxième site (CP) qui se trouve dans l'image de vaisseaux sanguins (VI) et correspond au premier site (DP),
dans lequel l'étape d'identification comprend l'identification, parmi des vaisseaux sanguins dans le tissu cérébral ou à proximité de celui-ci dans le sujet, du deuxième site (CP) qui est approprié pour que le dispositif de diagnostic d'épilepsie soit disposé pour détecter un signal électrique à partir du premier site (DP), sur la base des informations de fil, et
dans lequel, en référence à un tableau d'association qui est préalablement stocké dans une unité de stockage et dans lequel le premier site (DP) et un ou plusieurs sites dans les vaisseaux sanguins, qui correspondent au premier site (DP), sont associés l'un à l'autre, et en tenant compte d'un diamètre de vaisseau sanguin et de la taille du dispositif de diagnostic d'épilepsie, l'unité d'identification (33) identifie, parmi les un ou plusieurs sites où il est possible de disposer le dispositif de diagnostic d'épilepsie, un site qui est le plus proche en distance du premier site (DP) en tant que deuxième site (CP) ; et
une étape de commande d'affichage qui amène une unité d'affichage prédéterminée à afficher un élément indiquant le deuxième site (CP) dans l'image de vaisseaux sanguins (VI).

9. Programme informatique pour le diagnostic de l'épilepsie, amenant un ordinateur à exécuter :
une étape d'acquisition d'images consistant à acquérir une image de tissu cérébral (BI) et une image de vaisseaux sanguins (VI) d'un sujet à partir d'une machine d'acquisition d'images (IM) ;
une étape de réception consistant à recevoir l'identification d'un premier site (DP) qui se trouve dans l'image de tissu cérébral (BI) et est une cible à partir de laquelle un signal électrique doit être détecté ;
une étape de réception d'informations de fil et une étape de réception de dispositif consistant à recevoir des informations de fil, les informations de fil incluant une taille d'un dispositif de diagnostic d'épilepsie à disposer dans un vaisseau sanguin ;
une étape d'identification consistant à identifier, sur la base des informations de fil, un deuxième site (CP) qui se trouve dans l'image de vaisseaux sanguins (VI) et correspond au premier site (DP),
dans lequel l'étape d'identification comprend l'identification, parmi des vaisseaux sanguins dans le tissu cérébral ou à proximité de celui-ci dans le sujet, du deuxième site (CP) qui est approprié pour que le dispositif de diagnostic d'épilepsie soit disposé pour détecter un signal électrique à partir du premier site (DP), sur la base d'informations de fil, et
dans lequel, en référence à un tableau d'association qui est préalablement stocké dans une unité de stockage et dans lequel le premier site (DP) et un ou plusieurs sites dans les vaisseaux sanguins, qui correspondent au premier site (DP), sont associés l'un à l'autre, et en tenant compte d'un diamètre de vaisseau sanguin et de la taille du dispositif de diagnostic d'épilepsie, l'unité d'identification (33) identifie, parmi les un ou plusieurs sites où il est possible de disposer le dispositif de diagnostic d'épilepsie, un site qui est le plus proche en distance du premier site (DP) en tant que deuxième site (CP) ; et
une étape de commande d'affichage qui amène une unité d'affichage prédéterminée à afficher un élément indiquant le deuxième site (CP) dans l'image de vaisseaux sanguins (VI).
